# EUROPÄISCHE PATENTSCHRIFT

(11) **EP 0 590 374 B1**
(45) Veröffentlichungstag und Bekanntmachung des Hinweises auf die Patenterteilung: **06.12.1995**
(21) Anmeldenummer: 93114455.4
(22) Anmeldetag: 09.09.1993
(51) Int. Cl.: C01B 25/32, A61K 7/16

(54) **Verfahren zur Herstellung von Hydroxylapatit**
Process for preparation of hydroxyapatite
Procédé de préparation d'hydroxyapatite

(30) Priorität: 28.09.1992 DE 4232443
(43) Veröffentlichungstag der Anmeldung: 06.04.1994
(73) Patentinhaber: BK LADENBURG GmbH, Gesellschaft für chemische Erzeugnisse, D-68526 Ladenburg (DE)
(72) Erfinder: Maurer, Alexander Dr., D-68219 Mannheim (DE); Raab, Gudrun, D-69514 Laudenbach (DE); Raab, Günter, D-69514 Laudenbach (DE); Schmitt, Reinhold, D-69469 Weinheim (DE); Schober, Detlev, D-67227 Frankenthal (DE); Tänzler, Richard, Dr., D-69514 Laudenbach (DE)
(74) Vertreter: Grussdorf, Jürgen, Dr.

(56) Entgegenhaltungen:
- US-A- 4 274 879
- US-A- 4 324 772
- US-A- 4 849 193

## Beschreibung

Gegenstand der vorliegenden Erfindung ist ein neues Verfahren zur Herstellung von Hydroxylapatit.

In der Mundhygiene werden zur Herstellung von Zahnpasten die verschiedensten anorganischen Mineralien als Putzkörper zur Reinigung der Zähne verwendet. Es sind, um nur einige Beispiele zu nennen, Dicalciumphosphat, Calciumcarbonat, Siliciumdioxid, Maddrell'sches Salz, Aluminiumoxid und andere gebräuchlich. Von diesen Putzkörpern sind nicht alle aufgrund ihrer chemischen und ionischen Zusammensetzung unumstritten.

Wichtig für die Eignung eines Materials als Zahnputzkörper in einer Zahnpaste sind eine definierte Härte des Stoffes, eine sehr feine und eng definierte Kornstruktur, eine bestimmte Oberflächenbeschaffenheit und Absorptivität gegenüber Wasser/Glycerin-Mischungen.

Die Abrasivität von Zahnputzkörpern wird durch verschiedene Verfahren bestimmt (vgl. F.J. Dany: Seifen, Öle, Fette, Wachse, 104 (1978), S. 306-310). Die Abrasivität der erfindungsgemäßen Materialien wurden mit der Kupferplattenmethode bestimmt, jedoch bestehen zwischen den Analysemethoden bestimmte Korrelationen. Zur Erzielung einer sehr engen und reproduzierbaren Abrasivität sind drei Produktparameter wesentlich. Es muß auf eine bestimmte Kornstruktur (Mahlgrad), eine bestimmte Oberflächenstruktur auf dem Kristall/Agglomerat und eine hohe Kristallinität geachtet werden.

Die menschlichen Zähne bestehen weitgehend und vor allem der äußere Zahnschmelz aus Calciumphosphaten oder exakter aus Calciumhydroxylapatit hoher Reinheit. Naheliegend ist es daher, gefällten Calciumhydroxylapatit als Putzkörper zu verwenden, der eine weitgehende Identität mit dem natürlichen Zahnschmelz aufweist.

Es sind viele Methoden zur Fällung von Hydroxylapatit literaturbekannt (CA, B 1142-55). Der Hydroxylapatit fällt hierbei in nahezu allen Lösungen sehr feinkristallin. Die Kristalle neigen in Lösung und bei der anschließenden Isolierung des Feststoffes zum Agglomerieren. So zeigt sich, daß auf den üblichen Wegen durch Zudosierung einer Calciumquelle - wie Calciumhydroxid, Kalkmilch, Calciumcarbonat - in eine verdünnte Phosphorsäure oder in eine verdünnte Alkalimetallphosphatlösung oder umgekehrt von einer Phosphat/Phosphorsäurelösung in eine Kalkmilch auch unter eng gefaßten Fällungsparametern unter dem Gesichtspunkt einer definierten Abrasivität nur sehr unterschiedliche und kaum reproduzierbare Materialien produzierbar sind. Hinzu kommt, daß bei allen Fällungsverfahren von technischer Bedeutung Dicalciumphosphat als Nebenprodukt mitgefällt wird und sich aus dem Endprodukt nicht entfernen läßt. Dieses Material zeigt andere Kristallhärten und ist daher in dem erfindungsgemäßen Material gänzlich unerwünscht.

Aus der US-P 4 274 879 ist z.B. bekannt, Hydroxylapatit herzustellen, indem man Kalkmilch mit mindestens 60 % Phosphorsäure in stöchiometrischen Mengen bei Temperaturen von 80-85°C und einem pH-Wert der Reaktionslösung von ca. 9,0-11,0 in einer kontinuierlichen Reaktion zusammengibt. Die erhaltenen Produkte sind ungefähr sphärische Kristalle mit einem Durchmesser von 0,03 »m bis 0,5 »m und einem Hydroxylapatitgehalt von 98,5-99,5 %. Sie sind geeignet, um durch Sintern bei Temperaturen von 700°C Knochenersatzteile herzustellen. Als Zahnputzkörper sind sie aufgrund ihrer Feinheit nicht geeignet.

Aus der US-P 4 324 772 ist bekannt, in einem zweistufigen Prozeß zunächst Kalkmilch und Phosphorsäure bei 70-85°C und einem pH-Wert von 9,5-11, vorzugsweise bei 10,5, zusammenzugeben, um durch den Überschuß von Calciumhydroxid eine Bildung von Dicalciumphosphatverunreinigungen zu verhindern, und dann in einer zweiten Stufe durch Zugabe weiterer Phosphorsäure den pH auf 7,0-7,4 abzusenken, um überschüssiges Calciumhydroxid zu neutralisieren. Es entsteht ein Hydroxylapatit mit unter 1 »m Korngröße, welcher bei nicht exakter Verfahrensführung leicht Verunreinigungen von eingeschlossenem Ca(OH)₂ enthält. Als Zahnputzkörper ist dieses Produkt nicht geeignet.

Aus der US-P 4 849 193 ist ein weiteres Verfahren zur Herstellung von Hydroylapatit bekannt, bei dem zunächst eine saure Vormischung aus Phosphorsäure und Calciumhydroxidlösung hergestellt wird, die die ungefähre Zusammensetzung eines Monocalciumphosphats und einen pH-Wert von 1,5-3,5, vorzugsweise 2,0, aufweist. Diese wird bei 23°C in eine Vorlage von Calciumhydroxid langsam zudosiert, wobei ein pH-Wert von über 12 eingehalten wird, welcher erst bei Zugabe der Gesamtphosphorsäure auf 11-11,5 absinkt. Es wird ein feinteiliger Hydroxylapatit erhalten, der nach dem Sintern bei 1.000°C eine Korngröße von 0,2 »m hat und röntgenographisch frei von anderen Calciumphosphaten ist. Als Zahnputzkörper ist dieses Material nicht geeignet.

Aufgabe der vorliegenden Erfindung war es daher, einen Hydroxylapatit mit einer relativen Abrasivität nach der RDA-Methode nach Grabenstetter (vgl. F.J. Dany: Seifen, Öle, Fette, Wachse, 104 (1978), S. 306-310) von 110-150 herzustellen. Das nach dem beschriebenen Verfahren hergestellte Material soll diese Eigenschaft erfüllen und zudem eine hohe Kristallinität von einem Kristallisationsgrad von > 95 %, eine durch BET bestimmte Oberfläche von maximal 10 m²/g, vorzugsweise 0,1-5 m²/g, und eine mittlere Korngröße von 1-20 »m, vorzugsweise 4-8 »m, aufweisen.

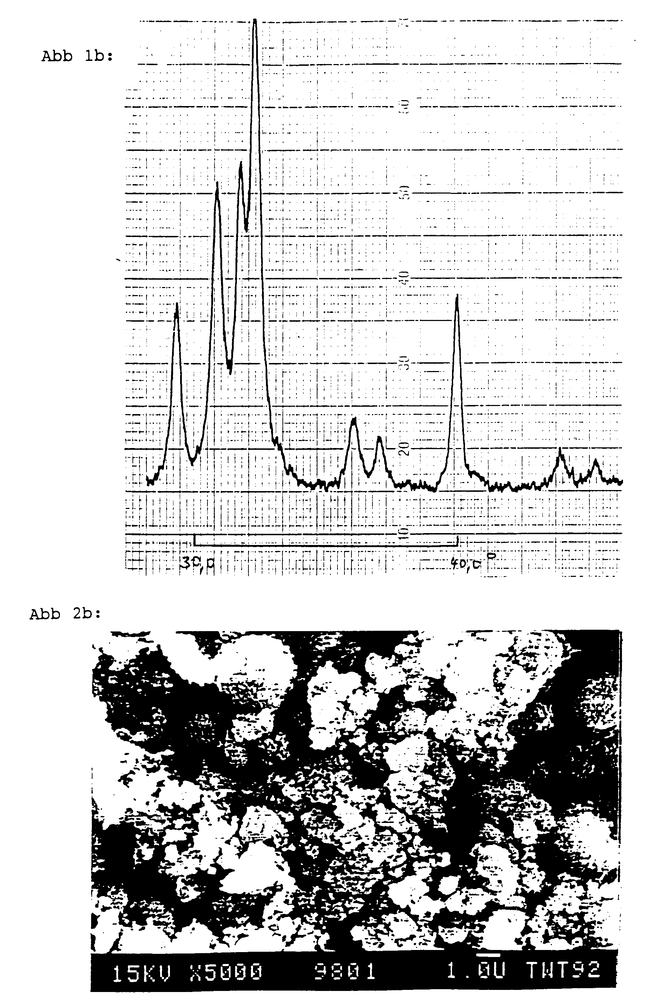

Völlig überraschend löst sich dieses Problem, wenn man eine saure Calciumphosphatlösung mit einem Ca:P-Mol-Verhältnis von 0,2-1 und bevorzugt 0,3-0,5 und Kalkmilch stöchiometrisch unter gleichzeitigem intensiven Mischen bei einem pH-Wert von 4-8, vorzugsweise 6,75-7,5, insbesondere 7,0-7,5, und einer Temperatur von 60-100°C, vorzugsweise 80-90°C, vereinigt. Es bilden sich Agglomerate mit feinen Aufwachsungen, die in elektronenrastermikroskopischen Aufnahmen (vgl. Abb. 2b) dargestellt sind. Diese bestehen aus einem Hydroxylapatit sehr hoher Kristallinität (> 95 % (vgl. Abb. 1b)) und die Fällungsprodukte sind frei von Dicalciumphosphatnebenprodukten, was überraschend ist, da man unter diesen Bedingungen (pH 7) auch Dicalciumphosphat ausfällt, sobald die stöchiometrischen Verhältnisse diesem entsprechen.

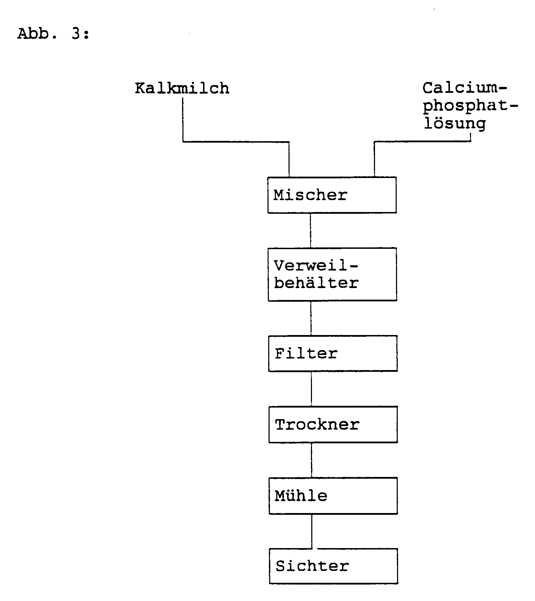

Das gesamte Verfahren ist in dem Fließschema (Abb. 3) beschrieben. Eine Calciumphosphatlösung und Kalkmilch werden im stöchiometrischen Verhältnis von Ca:P = 1,667 in ein kontinuierliches beheiztes Intensivmischaggregat geleitet. Es wird bei Temperaturen zwischen 60 und 100°C, vorzugsweise 80-90°C, gefällt. Der pH-Wert wird dabei auf ca. 7 eingestellt und bleibt bei stöchiometrischer Zugabe der Komponenten in einem Bereich von 5-8. Der Überlauf des Mischers wird in einen beheizten Rührbehälter geleitet, in dem unter mäßigem Rühren und Halten der Fällungstemperatur eine Nachreaktionsphase mit einer mittleren Verweilzeit bis zu 5 Stunden abgewartet wird. In der Regel reichen 1-2 Stunden mittlerer Verweilzeit aus, der Verlauf der Nachreaktion wird am Kristallisationsgrad des Endproduktes durch Röntgendiffraktogramm überwacht. Aus dem Verweilbehälter wird kontinuierlich Suspension abgezogen und zur Abtrennung des Hydroxylapatits einem üblichen Abtrennorgan, wie z.B. einem Bandfilter oder einer Zentrifuge, zugeleitet und dort gleichzeitig mit Wasser gewaschen und in einem Trockner das anhaftende Wasser verdampft. Eine anschließende Mahlung und Trocknung dient der Einstellung eines engen Kornbandes und der Zerstörung von bei der Trocknung auftretenden Agglomeraten. Man erhält so Partikel einer Größe von 1-20 »m, im Mittel 5-8 »m.

### Beispiele:

### Vergleichsbeispiel:

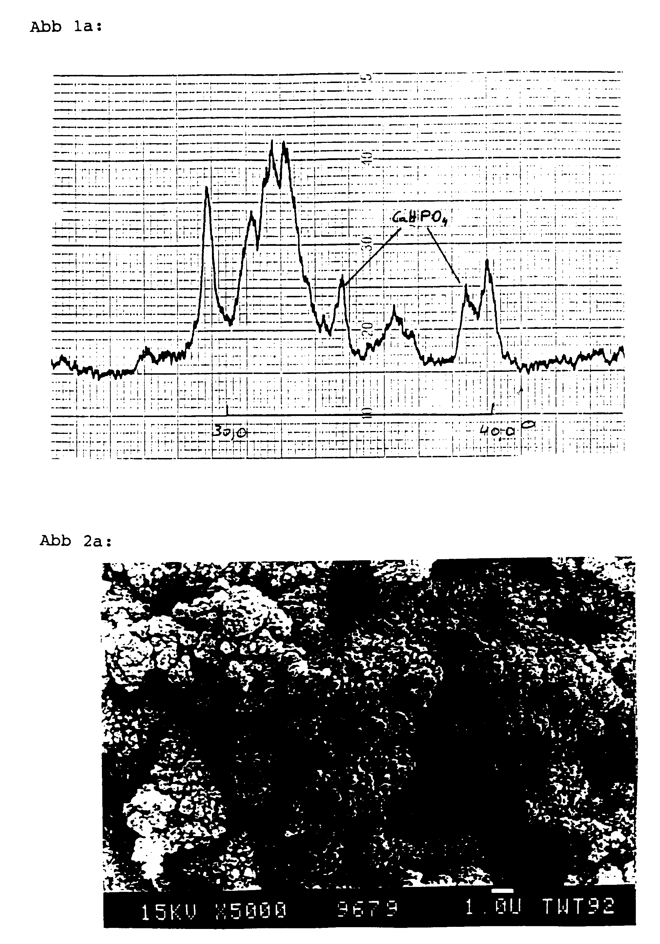

Über zwei miteinander gekoppelte Differenzialdosierwaagen werden eine 40 %-ige Phosphorsäure mit 146,5 kg/h und eine 20 %-ige Kalkmilch mit 380,8 kg/h (aus Calciumhydroxid mit einem Ca-Gehalt von 52,3 % und Deionat) in einen horizontalen Intensivdurchlaufmischer (Nutzinhalt 40 l), der auf eine Temperatur von 85°C vorgeheizt ist, dosiert. Die resultierende ca. 20 %-ige Suspension, welche einen pH-Wert von 7,2 aufweist, wird in einen 4m³-Rührreaktor geleitet, der auf 90°C gehalten wird. Eine Probe, die nach 3 h gezogen wurde, zeigt nach Abfiltration und Trocknung bei der röntgenspektrographischen Untersuchung ein Refraktogramm (Abb. 1a) mit deutlichen Dicalciumphosphatpeaks und bei einer elektronenmikroskopischen Aufnahme (Abb. 2a) eine inhomogene, feinkörnige Oberfläche. Korngröße ca. 0,8 »m.

### Beispiel 1:

In der im Vergleichsbeispiel beschriebenen Apparatur werden erfindungsgemäß eine Calcium-dotierte Phosphorsäure mit einem Ca-Gehalt von 3,0 % und einem P₂O₅-Gehalt von 28,8 % in einer Dosiergeschwindigkeit von 146,5 kg/h mit einer Kalkmilch (Ca-Gehalt: 10,8 %) in einer Dosiergeschwindigkeit von 328 kg/h bei 85°C umgesetzt. Der pH-Wert der Reaktionslösung liegt dabei zwischen 7 und 8. Es resultiert ein Dicalciumphosphatfreier Hydroxylapatit hoher Kristallinität nach Refraktogramm (Abb. 1b) und einer homogenen Oberflächenstruktur (Abb. 2b).

### Beispiel 2:

In einen auf 90°C vorgeheizten 50 l-Turbulentmischreaktor werden innerhalb von 90 min 26 kg einer Lösung mit einem Gehalt von 16,3 % P₂O₅ und 3,1 % Ca (hergestellt aus Kalk und Phosphorsäure) und 23,4 kg einer 25 %-igen Kalkmilch über Dosierpumpen in eine Wasservorlage von 5 l zugefahren. Die Mischung weist einen pH-Wert von 6,8 auf. Nach einer Nachreaktionszeit von 1 h wird der Slurry im Mischer auf einen Trockengehalt von 80 % unter Vakuumverdampfung aufkonzentriert. In einem diskoninuierlichen Wirbelbett wird das Produkt dann auf einen Feuchtegehalt < 2 % getrocknet und anschließend in einer Stiftmühle gemahlen. Man erhält einen Hydroxylapatit nach Beispiel 1 mit folgenden Eigenschaften:

| | |
|---|---|
| Schüttgewicht | 900 g/l |
| mittlere Korngröße | 6 »m |
| Abrasivität | 150 (Kupferplattentest) |

### Beispiel 3:

Über ein Differentialwägesystem werden kontinuierlich folgende Produktströme im Gewichtsverhältnis 1:1,4 und einem Gesamtmassestrom von 900 kg/h dosiert:
Komponente 1:
   Calciumphosphatlösung mit 24 % P₂O₅ und 3,5 % Ca
Komponente 2:
   25 %-ige Kalkmilch aus CaO und Wasser.

Als Fällungsapparatur wird ein 1.000 l diskoninuierlicher Turbulentmischreaktor mit Dampfheizung benutzt. Über einen Stutzen in 2/3 der Troghöhe wird mit einer Pumpe die Suspension, die einen pH-Wert von 6,5-7,8 aufweist, kontinuierlich in einen beheizten 4m³-Rührbehälter gepumpt. Aus diesem wird ab einem Füllgrad von 80 % kontinuierlich ein Strom von 750 l/h auf ein Bandfilter gegeben. Der filterfeuchte Kuchen wird in einem Stromtrockner getrocknet und in einer Sichtermühle auf die gewünschte Korngröße von 4-10 »m vermahlen.

### Beispiel 4:

Wie Beispiel 3 nur unter Benutzung eines 1 m³-Rührreaktors mit zwei auf einer Rührwelle angeordneten Trapezrührorganen als Umsetzungsreaktor und einem Gesamtmassestrom von 700 kg/h. Die Herstellung der Calciummonophosphatlösung erfolgt aus Calciumphosphatabfall und Phosphorsäure und anschließende Klarfiltration.

## Patentansprüche

1. Verfahren zur Herstellung eines als Zahnputzkörper geeigneten Hydroxylapatits, wobei man eine wässrige phosphorhaltige Lösung und eine Calciumsuspension in einem Molverhältnis von Ca:P = 1,667 gleichzeitig einem Intensivmischer zuführt und dort bei Temperaturen von 60 bis 100 °C unter Erhalt einer Hydroxylapatitsuspension umsetzt und das Hydroxylapatit aus der Suspension abtrennt, wäscht sowie trocknet, **dadurch gekennzeichnet**, daß man als phosphorsäurehaltige Lösung eine saure Calciumphosphatlösung mit einem Molverhältnis von Ca:P von 0,2:1 bis 1:1 einsetzt, und die Umsetzung bei einem pH-Wert von 4 bis 8 vornimmt.

2. Verfahren nach Anspruch 1, **dadurch gekennzeichnet**, daß man eine Calciumphosphatlösung mit einer Konzentration von 20 bis 50 Gew.-% einsetzt.

3. Verfahren nach Anspruch 1 und 2, **dadurch gekennzeichnet**, daß man eine Calciumhydroxidsuspension mit einer Konzentration von 5 bis 30 Gew.-% einsetzt.

4. Verfahren nach Anspruch 1 bis 3, **dadurch gekennzeichnet**, daß man die erhaltene Hydroxylapatitsuspension in einen Rührbehälter überführt und dort bis zu 5 Stunden bei 60 bis 90 °C nachreagieren läßt.

## Claims

1. Process for the production of a hydroxylapatite suitable as teeth cleaning material, whereby one passes an aqueous phosphoric acid-containing solution and a calcium suspension in a mol ratio of Ca:P = 1.667 simultaneously into an intensive mixer and there reacts at temperatures of 60 to 100°C with obtaining of a hydroxylapatite suspension and separates the hydroxylapatite from the suspension, washes, as well as dries, characterised in that, as phosphoric acid-containing solution, one uses an acidic calcium phosphate solution with a mol ratio of Ca:P of 0.2:1 to 1:1 and carries out the reaction at a pH value of 4 to 8.

2. Process according to claim 1, characterised in that one uses a calcium phosphate solution with a concentration of 20 to 50 wt.%.

3. Process according to claim 1 and 2, characterised in that one uses a calcium hydroxide suspension with a concentration of 5 to 30 wt.%.

4. Process according to claim 1 to 3, characterised in that one passes the hydroxylapatite suspension obtained into a stirrer container and there allows to post-react for up to 5 hours at 60 to 90°C.

## Revendications

1. Procédé pour la préparation d'une hydroxyapatite appropriée en tant que substance de nettoyage dentaire, dans lequel une solution aqueuse contenant du phosphore et une suspension de calcium dans un rapport molaire de Ca : P = 1,667 sont introduites simultanément dans un mélangeur intensif et elles y sont mises à réagir à des températures de 60 à 100 °C pour obtenir une suspension d'hydroxyapatite et l'hydroxyapatite est séparée de cette suspension, lavée et séchée, caractérisé en ce qu'on utilise en tant que solution contenant de l'acide phosphorique une solution de phosphate de calcium acide avec un rapport molaire de Ca : P de 0,2 : 1 à 1 : 1, et en ce qu'on effectue la réaction à un pH de 4 - 8.

2. Procédé selon la revendication 1, caractérisé en ce qu'on utilise une solution de phosphate de calcium avec une concentration de 20 - 50 % en poids.

3. Procédé selon la revendication 1 et 2, caractérisé en ce qu'on utilise une suspension d'hydroxyde de calcium avec une concentration de 5 - 30 % en poids.

4. Procédé selon l'une quelconque des revendications 1 à 3, caractérisé en ce qu'on transfère la suspension d'hydroxyapatite obtenue dans un récipient agité et que l'on l'y laisse réagir ultérieurement jusqu'à 5 heures à 60 à 90 °C.
